# EUROPEAN PATENT SPECIFICATION

(11) **EP 1 017 351 B1**
(45) Date of publication and mention of the grant of the patent: **30.11.2011**
(21) Application number: 98942225.8
(22) Date of filing: 24.08.1998
(51) Int. Cl.: A61H 31/00

(54) **RESUSCITATION DEVICE**
REANIMATIONSVORRICHTUNG
APPAREIL DE REANIMATION

(30) Priority: 27.08.1997 US 922723; 27.08.1997 US 924555
(43) Date of publication of application: 12.07.2000
(73) Proprietor: ZOLL Circulation, Inc., Sunnyvale CA 94085 (US)
(72) Inventor: MOLLENAUER, Kenneth, H., Portola Valley, CA 94028 (US); SHERMAN, Darren, R., Portola Valley, CA 94028 (US); BYSTROM, Steven, R., Portola Valley, CA 94028 (US); MINER, Cameron, Portola Valley, CA 94028 (US)
(74) Representative: Viering, Jentschura & Partner
(86) International application number: PCT/US1998/017502
(87) International publication number: WO 1999/009929

(56) References cited:
- WO-A-94/26229
- FR-A- 923 379
- US-A- 651 962
- US-A- 4 004 579
- US-A- 4 770 164

## Description

### Background of the Invention

Cardiopulmonary resuscitation (CPR) is a well known and valuable method of first aid. CPR is used to resuscitate people who have suffered from cardiac arrest after heart attack, electric shock, chest injury and many other causes. During cardiac arrest, the heart stops pumping blood, and a person suffering cardiac arrest will soon suffer brain damage from lack of blood supply to the brain. Thus, CPR requires repetitive chest compression to squeeze the heart and the thoracic cavity to pump blood through the body. Very often, the victim is not breathing, and mouth to mouth artificial respiration or a bag valve mask is used to supply air to the lungs while the chest compression pumps blood through the body.

It has been widely noted that CPR and chest compression can save cardiac arrest victims, especially when applied immediately after cardiac arrest. Chest compression requires that the person providing chest compression repetitively push down on the sternum of the victim at 80-100 compressions per minute. CPR and closed chest compression can be used anywhere, wherever the cardiac arrest victim is stricken. In the field, away from the hospital, it may be accomplished by ill-trained by-standers or highly trained paramedics and ambulance personnel.

When a first aid provider performs chest compression well, blood flow in the body is typically about 25-30% of normal blood flow. This is enough blood flow to prevent brain damage. However, when chest compression is required for long periods of time, it is difficult if not impossible to maintain adequate compression of the heart and rib cage. Even experienced paramedics cannot maintain adequate chest compression for more than a few minutes. Hightower, et al., Decay In Quality Of Chest Compressions Over Time, 26 Ann. Emerg. Med. 300 (Sep. 1995). Thus, long periods of CPR, when required, are not often successful at sustaining or reviving the victim. At the same time, it appears that, if chest compression could be adequately maintained, cardiac arrest victims could be sustained for extended periods of time. Occasional reports of extended CPR efforts (45-90 minutes) have been reported, with the victims eventually being saved by coronary bypass surgery. See Tovar, et al., Successful Myocardial Revascularization and Neurologic Recovery, 22 Texas Heart J. 271 (1995).

In efforts to provide better blood flow and increase the effectiveness of bystander resuscitation efforts, modifications of the basic CPR procedure have been proposed and used. Of primary concern in relation to the devices and methods set forth below are the various mechanical devices proposed for use in main operative activity of CPR, namely repetitive compression of the thoracic cavity.

The device shown in Barkolow, Cardiopulmonary resuscitator Massager Pad, U.S. Patent 4,570,615 (Feb. 18, 1986), the commercially available Thumper device, and other such devices, provide continuous automatic closed chest compression. Barkolow and others provide a piston which is placed over the chest cavity and supported by an arrangement of beams. The piston is placed over the sternum of a patient and set to repeatedly push downward on the chest under pneumatic power. The victim must first be installed into the device, and the height and stroke length of the piston must be adjusted for the patient before use, leading to delay in chest compression. Other analogous devices provide for hand operated piston action on the sternum. Everette, External Cardiac Compression Device, U.S. Patent 5,257,619 (Nov. 2, 1993), for example, provides a simple chest pad mounted on a pivoting arm supported over a patient, which can be used to compress the chest by pushing down in the pivoting arm. These devices are not clinically more successful than manual chest compression. See Taylor, et al., External Cardiac Compression, A Randomized Comparison of Mechanical and Manual Techniques, 240 JAMA 644 (Aug. 1978). Other devices for mechanical compression of the chest provide a compressing piston which is secured in place over the sternum via vests or straps around the chest. Woudenberg, Cardiopulmonary Resuscitator, U.S. Patent 4,664,098 (May 12, 1987) shows such a device which is powered with an air cylinder. Waide, et al., External Cardiac Massage Device, U.S. Patent 5,399,148 (Mar. 21, 1995) shows another such device which is manually operated. In another variation of such devices, a vest or belt designed for placement around the chest is provided with pneumatic bladders which are filled to exert compressive forces on the chest. Scarberry, Apparatus for Application of Pressure to a Human Body, U.S. Patent 5,222,478 (Jun. 29, 1993) and Halperin, Cardiopulmonary Resuscitation and Assisted Circulation System, U.S. Patent 4,928,674 (May 29, 1990) show examples of such devices.

Several operating parameters must be met in a successful resuscitation device. Chest compression must be accomplished vigorously if it is to be effective. Very little of the effort exerted in chest compression actually compresses the heart and large arteries of the thorax and most of the effort goes into deforming the chest and rib cage. The force needed to provide effective chest compression creates risk of other injuries. It is well known that placement of the hands over the sternum is required to avoid puncture of the heart during CPR. Numerous other injuries have been caused by chest compression. See Jones and Fletter, Complications After Cardiopulmonary Resuscitation, 12 AM. J. Emerg. Med. 687 (Nov. 1994), which indicates that lacerations of the heart, coronary arteries, aortic aneurysm and rupture, fractured ribs, lung herniation, stomach and liver lacerations have been caused by CPR. Thus the risk of injury attendant to chest compression is high, and clearly may reduce the chances of survival of the victim vis-à-vis a resuscitation technique that could avoid those injuries. Chest compression will be completely ineffective for very large or obese cardiac arrest victims because the chest cannot be compressed enough to cause blood flow. Chest compression via pneumatic devices is hampered in its application to females due to the lack of provision for protecting the breasts from injury and applying compressive force to deformation of the thoracic cavity rather than the breasts.

CPR and chest compression should be initiated as quickly as possible after cardiac arrest to maximize its effectiveness and avoid neurologic damage due to lack of blood flow to the brain. Hypoxia sets in about two minutes after cardiac arrest, and brain damage is likely after about four minutes without blood flow to the brain, and the severity of neurologic defect increases rapidly with time. A delay of two or three minutes significantly lowers the chance of survival and increases the probability and severity of brain damage. However, CPR and ACLS are unlikely to be provided within this time frame. Response to cardiac arrest is generally considered to occur in four phases, including action by Bystander CPR, Basic Life Support, Advanced Life Support, and the Emergency Room. By-stander CPR occurs, if at all, within the first few minutes after cardiac arrest. Basic Life Support is provided by First Responders who arrive on scene about 4-6 minutes after being dispatched to the scene. First responders include ambulance personnel, emergency medical technicians, fireman and police. They are generally capable of providing CPR but cannot provide drugs or intravascular access, defibrillation or intubation. Advanced Life Support is provided by paramedics or nurse practitioners who generally follow the first responders and arrive about 8-15 minutes after dispatch. ALS is provided by paramedics, nurse practitioners or emergency medical doctors who are generally capable of providing CPR, drug therapy including intravenous drug delivery, defibrillation and intubation. The ALS providers may work with a victim for twenty to thirty minutes on scene before transporting the victim to a nearby hospital. Though defibrillation and drug therapy is often successful in reviving and sustaining the victim, CPR is often ineffective even when performed by well trained first responders and ALS personnel because chest compression becomes ineffective when the providers become fatigued. Thus, the initiation of CPR before arrival of first responders is critical to successful life support. Moreover, the assistance of a mechanical chest compression device during the Basic Life Support and Advanced Life Support stages is needed to maintain the effectiveness of CPR.

US 4 770 164 A discloses a resuscitation apparatus for resuscitating a patient having cardiocirculatory arrest comprising a supple band that is passed around the thorax.

US 65/962 A discloses an apparatus for treating respiratory diseases.

### Summary

The devices described below provide for circumferential chest compression with a device which is compact, portable or transportable, self-powered with a small power source, and easy to use by by-standers with little or no training. Additional features may also be provided in the device to take advantage of the power source and the structural support board contemplated for a commercial embodiment of the device.

The present invention provides a chest compression device having the technical features as described in claim 1.

Preferred embodiments of the present invention are described in the dependent claims.

In its simplest form, the device includes a broad belt which wraps around the chest and is buckled in the front of the cardiac arrest victim. The belt is repeatedly tightened around the chest to cause the chest compression necessary for CPR. The buckles and/or front portion of the belt are anatomically accommodating for the female breast, or for the obese person, so that the device is effective for women as well as men. The buckle may include an interlock which must be activated by proper attachment before the device will activate, thus preventing futile belt cycles. The operating mechanism for repeatedly tightening the belt is provided in a support board, and comprises a rolling mechanism which takes up the intermediate length of the belt to cause constriction around the chest. The roller is powered by a small electric motor, and the motor powered by batteries and/or standard electrical power supplies such as 120V household electrical sockets or 12V DC automobile power sockets (car cigarette lighter sockets). (An initial prototype used a power drill with a single 9.6V rechargeable battery, and provided powerful chest compression for about ten minutes.) The batteries and any necessary transformers may be housed in the support board, and the support board may be made in sizes useful for supporting the victim's head, adequate for storing batteries and other accessories, and convenient for mounting within office buildings, factories, airplanes and other areas of potential need. Thus, numerous inventions are incorporated into the portable resuscitation device described below.

The portable resuscitation device may incorporate a number of features and accessories that aid in the administration of CPR and other therapy. By-standers may be unable to confidently determine if chest compression is needed, or when it should be stopped. Accordingly, the device may be combined with an interlock system including a heart monitor or EKG which diagnoses the condition of the patient, and circuitry or a computer which initiates, permits or forbids belt operation accordingly. The power supply provided for belt constriction may also be used to provide power for defibrillation (an appropriate treatment for many cardiac arrests). Again, bystanders will most likely not be capable of determining when defibrillation is appropriate, and the defibrillation portion of the device may be provided with an interlock system including the heart monitor or EKG which diagnoses the condition of the patient and circuitry which initiates, permits, or forbids defibrillation. Expert systems implemented through the circuitry or computer modules can accomplish these functions.

Automatic, computer driven therapy of this nature may provide early and appropriate life saving response to many cardiac arrest patients who would otherwise die. However, some situations in which the device might be used may call for expert supervision of the CPR process by emergency medical technicians, emergency room doctors, or cardiologists. To this end, the expert systems mentioned above may be replaced with the expert diagnosis and decision-making of medical personnel through a telemetry system housed within the support board of the device. The support board can include a telemetry system which automatically dials medical personnel in a nearby hospital, emergency medical crew, ambulance, or even a central diagnostic and control facility. Interlocks, limit switches and other typical sensors can be used to sense the proper position and closure of the belt about the chest of the patient. Heart monitors and EKG electrodes can sense the heart rate and EKG of the victim. Using communication equipment within the device, this information can be communicated from the device to medical personnel remote from the victim. Through the same system, the medical personnel can communicate the device to initiate, permit or prohibit belt constriction or defibrillation, as dictated by preferred medical procedures. Communication can be established through normal telephone lines and a cordless telephone, or through a cellular telephone system, paging system, internet or any other communications system. The device can be programmed with location information, or provided with GPS capabilities to determine the location of the device, and this information can be automatically transmitted to an emergency response system such as the 911 system when the system is placed in use.

### Brief Description of The Drawings

Figure 1 is an overview of the resuscitation device, showing the inner and outer vests partially open.
Figure 2 is an overview of the resuscitation device in the buckled configuration.
Figure 3 is a detail view of the buckle used to close the device about a victim.
Figure 4 shows the spool assembly used to operate the compression belt.
Figure 5 shows an alternative embodiment of the spool assembly used to operate the compression belt.
Figure 6 is a view of the resuscitation device properly positioned on a victim.
Figure 7 shows the resuscitation device fitted with a number of additional devices for use during resuscitation.
Figure 8 shows a detail view of the CRP module of Figure 7.
Figure 9 shows a detail view of the defibrillation module of Figure 7 useable together with the device of the present invention.
Figure 10 shows a detail view of the airway management module of Figure 7 useable together with the device of the present invention.
Figure 11 shows a detail view of the control and communications module of Figure 7.
Figure 12 shows a block diagram of the communications system.
Figure 13 is a block diagram of the motor control system.

### Detailed Description of the Invention

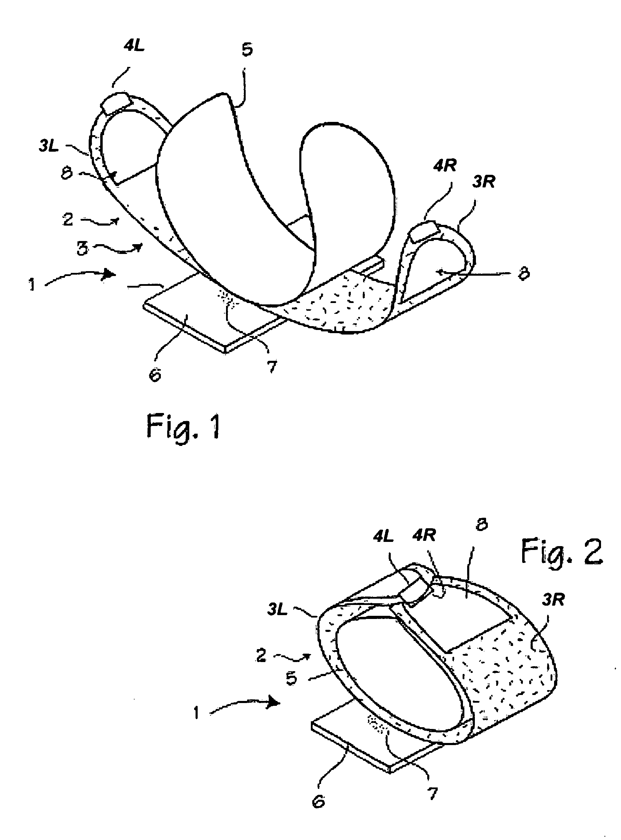

Figure 1 shows a simplified version of the resuscitation device 1. The mechanisms used for compressing the chest includes compression assembly 2 which includes a chest compression belt 3 with buckles **41** and **4r,** a friction liner 5, a support board 6 and a motor driven spool assembly 7. The support board 6 is placed under a cardiac arrest victim, and the compression belt 3 and friction liner 5 are wrapped around the victim's chest. The chest compression belt, having a left side 31 and a right side **3r,** is buckled over the victims chest by latching the buckles **4l** and **4r** together. In this configuration, the friction liner 5 will fit between the chest compression belt 3 and the victim and any clothes worn by the victim. The compression belt may be made of any strong material, and sail cloth has proven adequate for use.

The compression belt may also be referred to as a vest, corset, girdle, strap or band. The friction liner may be made of Teflon®, tyvek™ or any other low friction material (by low friction, we mean a material that will permit sliding of the compression belt with less friction than expected between the belt and the victims clothing or bare skin). The friction liner may be made with any suitable lining material, as its purpose is to protect the victim from rubbing injury caused by the compression belt, and it may also serve to limit frictional forces impeding the compression belt operation. The friction liner can be provided in the form of a belt, vest, corset, girdle, strap or band, and may partially or completely encircle the chest.

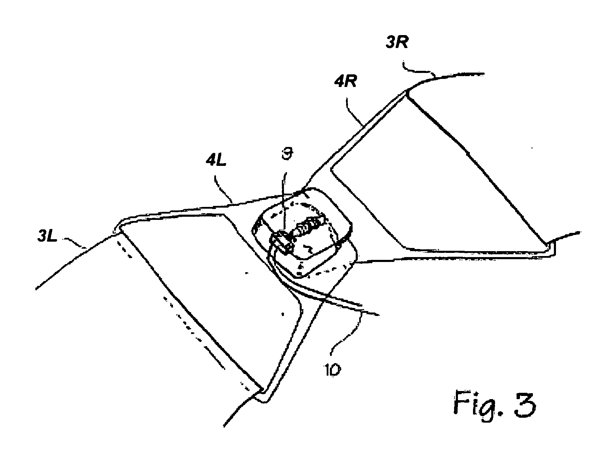

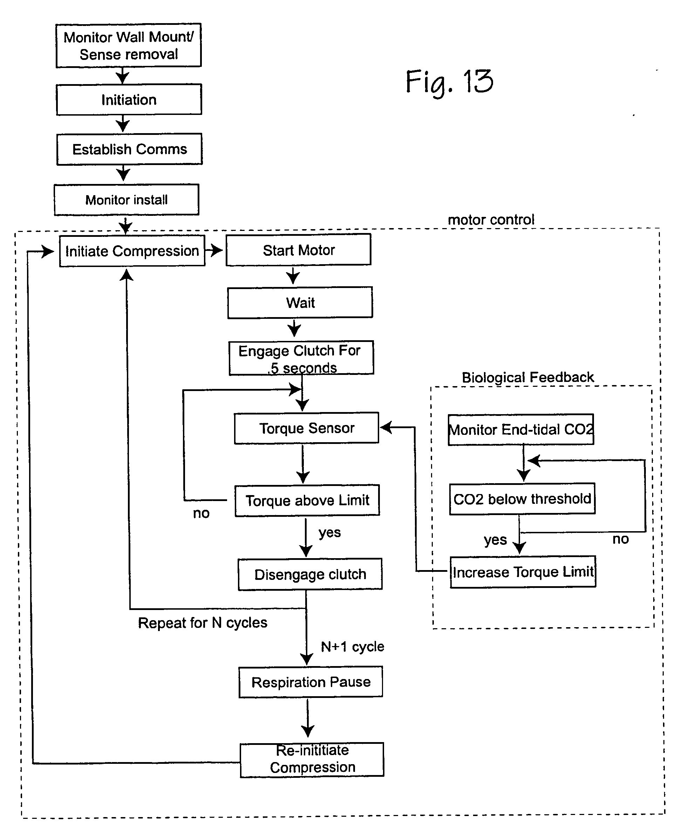

The front of the compression belt 3, including the buckles **41** and **4r,** are configured to provide a broad pressure point over the sternum of the victim. This is illustrated in Figure 2. Large openings **8** may be provided to accommodate female breasts and obese male breasts. The underside of the buckles **4l** and **4r** are smooth and broad, to distribute compressive force evenly over a wide area of the chest corresponding to the sternum. The point at which the buckle attaches to the chest compression belt may vary considerably, from the front of the chest to the back of the compression assembly, and the openings 8 may be provided in the buckles rather than the belt itself. Figure 3 shows a detail of the buckles 4 used to fasten the compression belt about the chest of the victim. The buckle may be of any type, and preferably includes a latch sensing switch **9** operably connected through wire **10** to the motor control system (see Figure 13) to indicate that the device has been buckled about the victims chest and is ready for the initiation of compression cycles. The buckles shown in Figure 3 are D-ring shaped buckles with large openings 8, attached to the compression belt 3. Other fasteners and fastening means may be used.

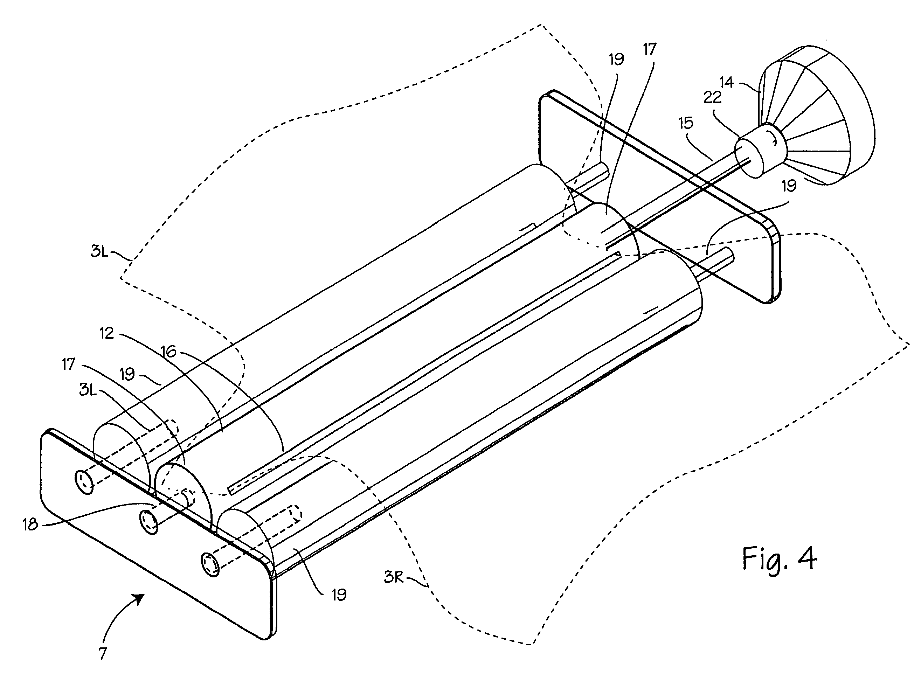

The chest compression belt 3 is repeatedly tightened about the chest of a victim through the action of one or more tightening spools which make up the spool assembly **7** located within the support board **6.** The spool assembly, illustrated in Figure 4, includes at least one spool or reel connected to the compression belt **3** at the back of the belt, preferably near the center or saggital line of the compression belt (although it may be located on the front or side of compression belt). Figure 4 shows a view of the spool assembly and its attachment to the compression belt. A spool assembly includes a single drive spool **12** operably connected to the motor **14** through drive shaft **15.** The compression belt is secured to the drive spool in any suitable manner. In this case a longitudinal slot **16** is provided in the drive spool **12.** The slot extends radially or chordally through the drive spool, and extends axially for a length corresponding to the width of the compression belt, leaving the ends 17 solid for connection to the drive shaft **15** and journal shaft **18.** The belt is slipped through the slot to create a secure connection between the belt and the drive spool. When secured in this manner, the rotation of the drive spool **12** will take up the right side of the compression belt **3r** and the left side of the compression belt **31** and roll them up onto the spool, thus tightening the compression belt about the chest of the victim wearing the device. Spindles or alignment rollers **19** provide for alignment and low friction feed of the belt onto the roll created by operation of the drive shaft.

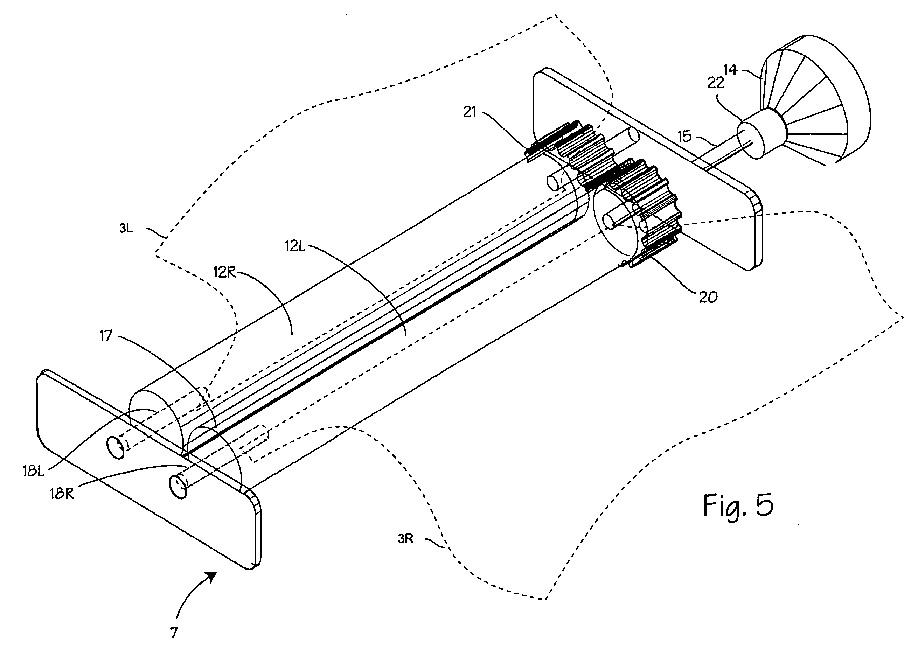

Many alternative embodiments can be envisioned for the rolling mechanism, and one such alternative is illustrated in Figure 5. Spools **12l** and **12r** are aligned in parallel and interconnected by a transmission gear **20** and planetary gear **21** and journaled upon shafts **18l** and **18r.** The drive shaft **15** is attached to spool **12r** (or spool **12l**) and operably attached to motor **14.** The motor turns the shaft **18r** and spool **12r** in a counterclockwise direction to pull the right side of the compression belt **3r** to the left and roll onto the spool. The transmission gear **20** acts upon the planetary gear **21** to cause clockwise rotation of spool **12l**, which in turn pulls and wraps the left side of the compression belt **31** onto the spool **12l.**

Thus, many embodiments of mechanisms which can cause repeated cyclic tightening of the compression vest about the chest of the victim may be envisioned. The compression belt serves to radially compress the chest through the cooperative action of the belt, board, and buckle, and to disperse the compressive force around the chest.

The motor is energized to rotate the spools and cause the compression belt to constrict around the chest of a victim. A motor such as a battery operated hand drill motor provides adequate chest compression for the purposes of CPR. To cause repetitive constriction of the compression belt 3, the motor 14 must be attached via a clutch **22** or other such mechanism. The motor 14 may be attached to the drive shaft 15 through a torque slipping clutching mechanism which engages the drive shaft until a high torque is achieved (indicating great resistance to further constriction, and thus indicating that the victim's chest has been compressed), and releases automatically upon such high torque, only to re-engage after the belt has been expanded in response to the normal elastic expansion of the victim's chest. In this manner, repetitive compression is achieved without need to repeatedly energize and de-energize the motor, thereby extending the length of operating time for any given battery supply. Alternatively, the motor may be repeatedly energized and de-energized, with the spools spinning freely during periods in which the belt is de-energized, wherein the clutch mechanism 22 will be similar to clutch mechanisms used on electric drills (which engage during operation of the drill but spin freely when the drill is de-energized). While the natural elastic expansion of the chest should make it unnecessary to drive the belt toward a loose condition, positive loosening may be achieved by reversing the motor or reversing the action of the motor through appropriate clutch or gear mechanisms. Timing of compressions is regulated through a computer module or a simple relay (windshield wiper style relays), and preferably will conform to standard of the Advanced Cardiac Life Support guidelines or Cardiopulmonary Resuscitation guidelines, or any other medically acceptable resuscitation regime. Current guidelines put forth by the American Heart Association call for 60-100 chest compressions per minute.

The motor is preferably battery powered, with provisions for taking power from any available power source. Batteries **23** may be stored within the support board 6. Three volt batteries of convenient size, already available for use with numerous power tools, provide about five minutes of compression per battery, while twelve volt batteries (1700mA-h per battery) have provided about ten minutes of compression per battery. A thirty minute total battery capacity is desirable (corresponding to the estimated average time between cardiac arrest and transport to the hospital). Accordingly, several batteries may be installed within the support board and electrically connected to the motor and its controller. The batteries are provided with a trickle charge through a charger socket and charger plugged into 120V AC power when the device is not in use. (It is intended that the device be installed in factories, office buildings, airplanes and other facilities with relatively stable sources of power, and that the unit remain plugged in and charging when not in use.) If AC power is readily available at the site of use, the device may continue to run on AC power to preserve the batteries for later use. The unit may also be plugged into an automobile power jack with an appropriate auto adapter, thus providing for use where an automobile is the only source of power, and for extended use in an ambulance.

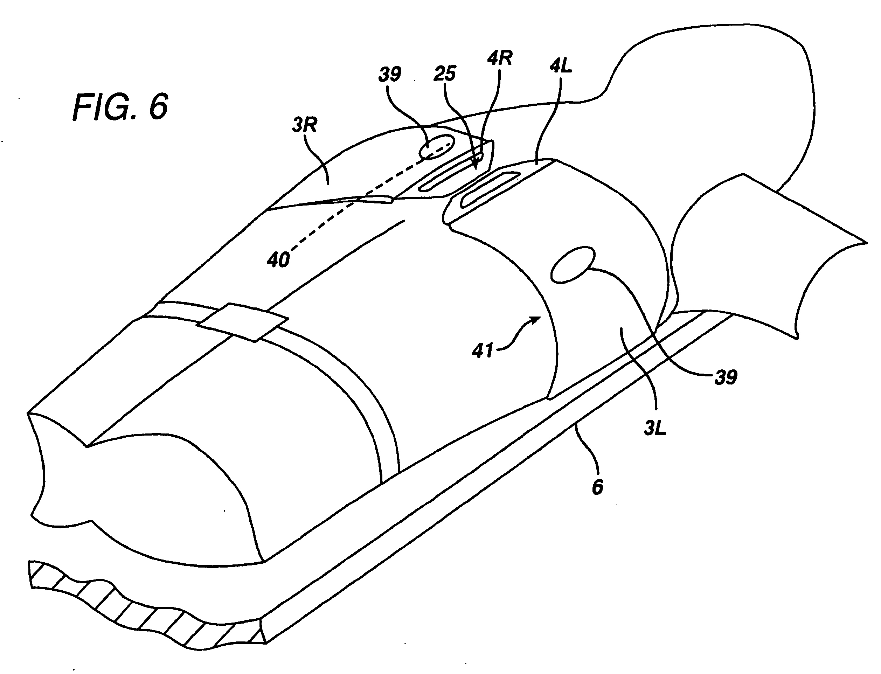

Figure 6 shows the resuscitation device installed on a cardiac arrest victim. The support board is placed under the victim, and the right and left portions of the compression belt are wrapped around the victim's chest and buckled over the front of the chest, indicated by arrow 25. Once in place, the system may be put into operation by manually starting the motors or by automatic initiation given the proper feedback from sensors located on the device, including the buckle latch sensors.

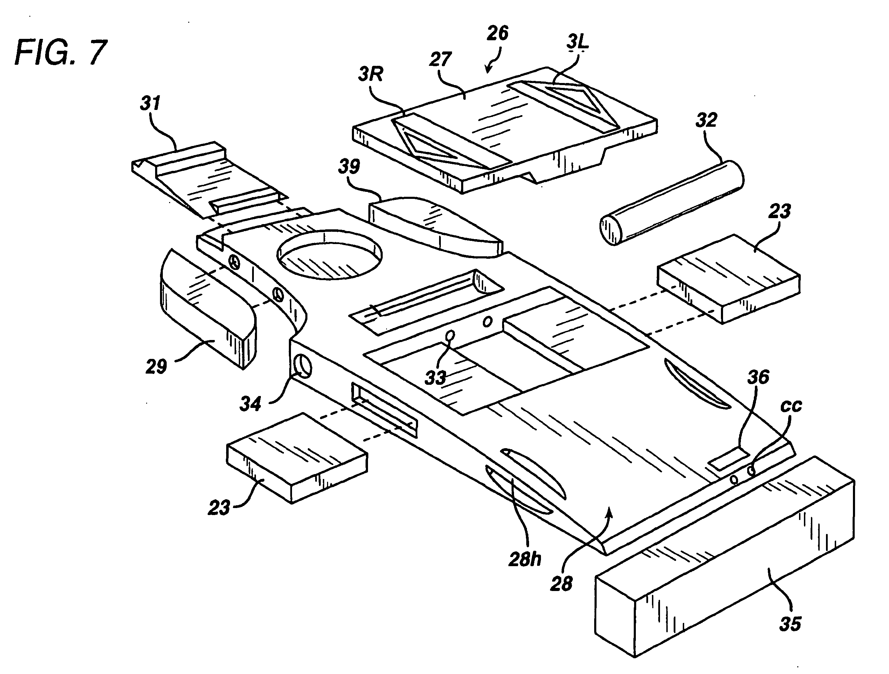

A number of features may be combined with the basic system described above. The structure necessary for housing the operating mechanism for the belt, referred to as the support board above, can serve also as storage for additional devices used during resuscitation. Figure 7 illustrates the resuscitation device 1 in a potential commercial embodiment. The support board 6 is sized to reach approximately from the lower lumbar region to the shoulders of a victim. The compression module **26** is separable from the support board 6, and includes the compression belt and friction vest stored within the compression module. The spool assembly and motor are also stored within the compression module, although the motor may also be installed in the support board. In this figure, the compression module comprises a small support board **27** which fits into the larger system support board **28.** Taking advantage of available space in the system support board, a compartment **29** for storage of airway management devices (bag masks, oxygen masks, etc.), and a compartment **30** for storage of defibrillation equipment (electrodes and paddles, etc.) are included with the support board. A control and communication module **31** may also be incorporated into the support board. A small oxygen bottle **32** may be included, along with hoses routed to an accessible point on the board, and any connector desired for connection between the oxygen bottle and devices provided in the airway management compartment. Batteries 23 are stored within the support board (the number of the batteries chosen according the desired operating time, and the placement of the batteries dictated by available space). Batteries are operably connected to the motor in the compression module through electrical connectors **33** and appropriate wiring throughout the support board. The batteries can also be operably connected to the defibrillation module and control and communications module. Although long life batteries can be used, rechargeable batteries may be preferred. Accordingly, charging connection **34** on the support board is provided for charging the batteries or operating the device through outside power supplies.

The device is intended to be stored for long periods of time between uses, and storage holder **35** is provided for this purpose. The storage holder can include such necessities as power supply connectors, a power plug, a charging transformer. A removal sensor **36** is included in the support board to sense when the support board is removed from the storage holder (which, as described below, can be used as a condition indicating use of the device, and therefore the need to alert emergency medical personnel). The removal sensor can comprise a simple limit switch which senses physical removal of the system, and the limit switch can be used as a power switch or awaken switch which starts initiation of the system. The removal sensor can comprise a current sensor on the charging lines cc which treat cessation of charging current, increase in current draw through the charging system, or motor current as an indication of use. The choice of sensor may be made with many practical considerations in mind, such as the desire to avoid treating power outages as indications of use and other such unintended initiations. The state in which the device is deemed to be "in use" can be chosen according to the practical considerations, and in most instances it is expected that mere removal of the resuscitation device from the holder will constitute a clear signal someone has determined that a victim requires its use, and that emergency medical personnel should be dispatched to the location of the device. There are some environments in which later conditions will be used to indicate that the device is "in use," such as when installed in ambulances, airplanes, hospitals or other environments where it might be advisable to remove the device from its storage holder as a precaution or preparatory measure, and delay initiation of communications until the device is deployed or installed on the victim. In such cases, the buckle latch shown in Figure 3 can be used as the sensor that indicates that the resuscitation device is in use.

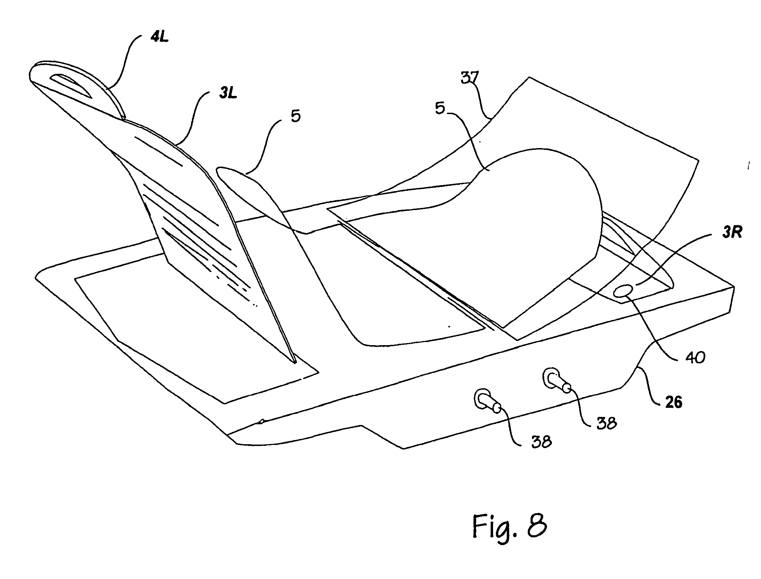

Figure 8 shows the details of the compression module **26.** When not in use, the module is covered with a tear sheet **37** which protects the compression belt from wear. The buckles are readily visible under the tear sheet. The electrical connectors **38** connect the batteries in the support board with the motor inside the compression module. The inside of the compression belt is fitted with penetrating electrodes **39** in the right sternum parasaggital location **40** and left rib medial location **41** for establishing the electrode contact needed for EKG sensing. These electrodes may be dispensed with in environments where proper placement of the defibrillation electrodes can be assumed due to a high level of training amongst likely bystanders and first responders. The friction vest 5 is secured to the compression module above the spool assembly location.

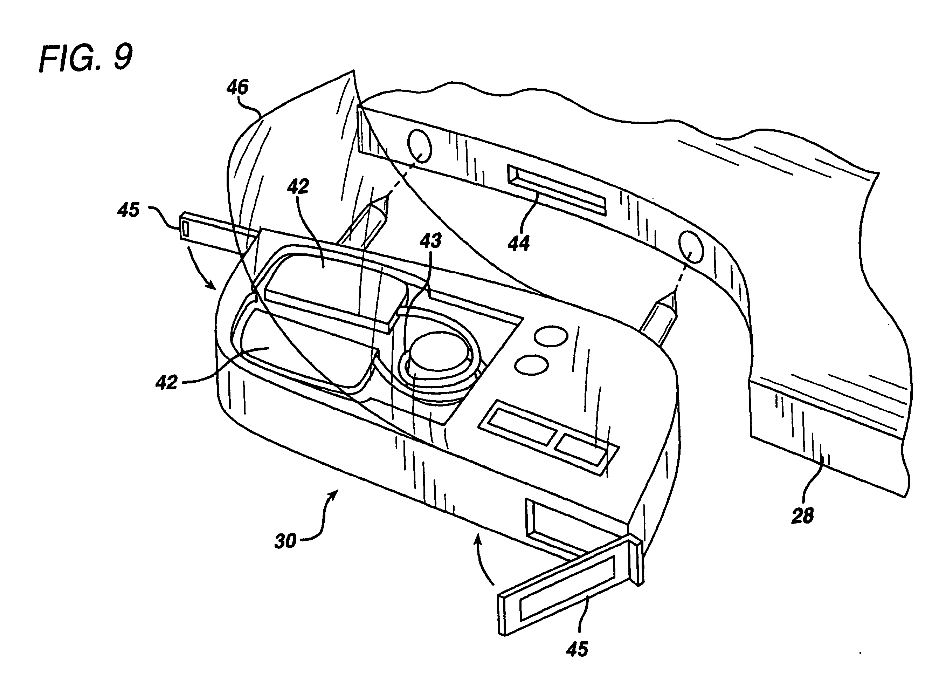

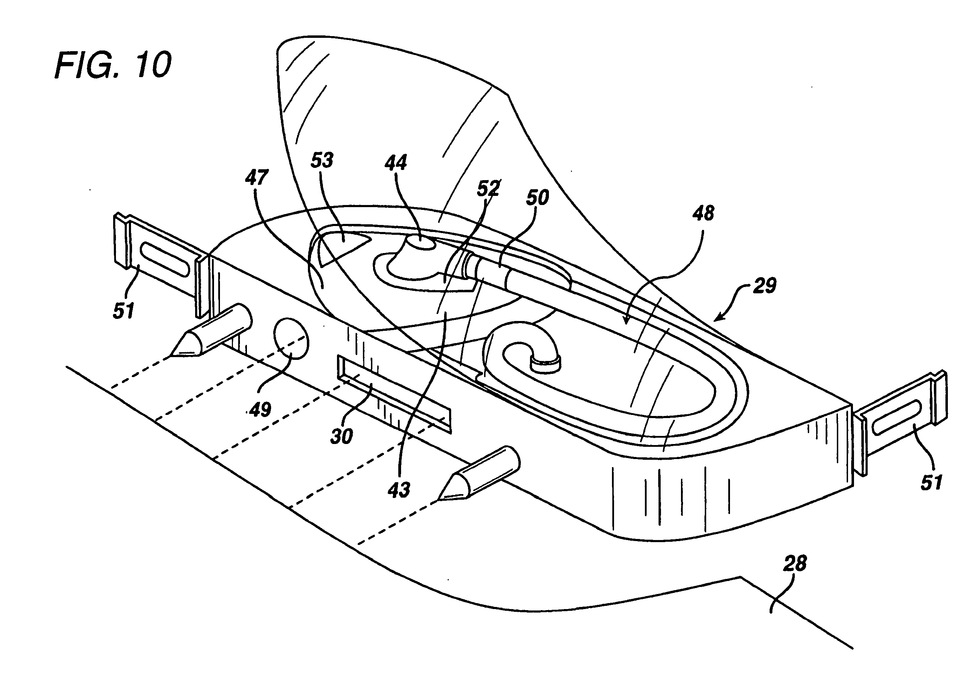

Figure 9 shows a detail view of the defibrillation module 30. The defibrillation module includes a pair of defibrillation electrodes **42** connected to the batteries through the power connections **43.** The defibrillation electrodes will be controlled by circuitry housed within the defibrillation module, and may be connected to the control module through the data port **44.** The defibrillation module is releasably attached to the support board 28 with quick release latches **45.** Tear sheet **46** protects the components of the defibrillation module during storage and provides ready access for use. Figure 10 shows the detail view of the airway management module 29, which includes an oxygen mask **47,** a length of tubing **48** and an air fitting **49** connecting the oxygen mask to the oxygen bottle within the support board. The oxygen mask serves as a blood gas exchange means, supplying oxygen to the lungs for exchange with blood gas such as CO₂. Optional medicine injectors **50** may be operably connected to the masks or hose to provide for automatic injection of ACLS medications into the airway. The defibrillation module is releasably attached to the support board 28 with quick release latches **51.** Tear sheet 46 protects the components of the airway management module during storage and provides ready access for use. An end-tidal CO₂ monitor **52** can be included in the mask to provide for biological feedback and monitoring of the success of the CPR. A skin mounted blood oxygen level monitor **53** can also be mounted on the mask for the same purpose (fingertip blood oxygen sensors may also be used, and supplied in the overall assembly to be readily available). The biological data obtained by the sensors is transmitted to the control module via appropriate wiring in the mask and support board.

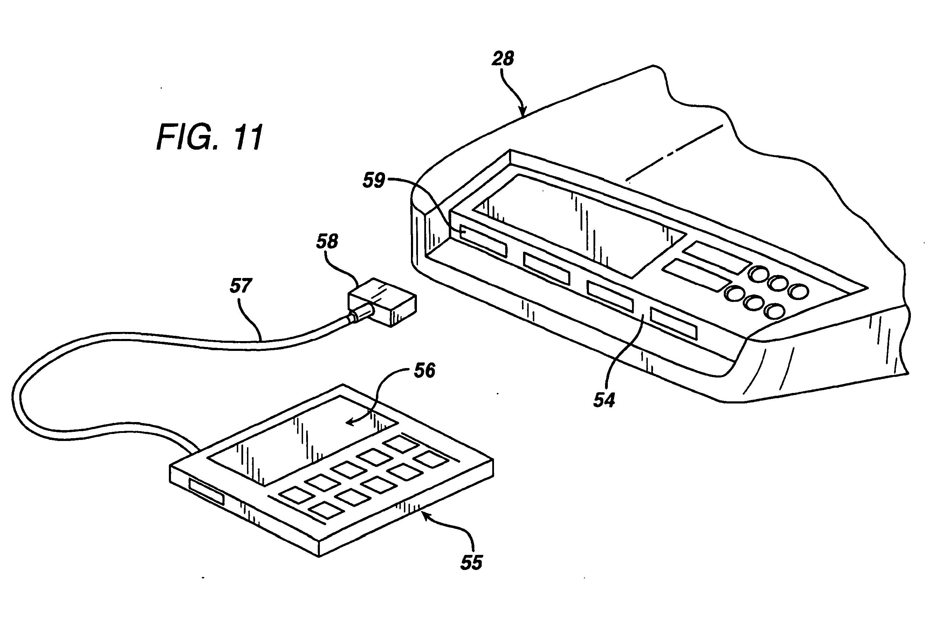

Figure 11 shows a detail view of the control and communications module. The control unit **54** is connected to the compression module, defibrillation module and the airway management module through appropriate wiring through the support board. The control unit is optionally connected to the communications unit **55.** The communications unit includes means for communicating the EKG and other measured medical parameters sensed on the board to the screen **56** and via telephone to remote medical personnel. The communications unit can include a telephone handset or speaker phone. Because the device is most likely to be used at a location separate from the storage holder, the communications module preferably includes a wireless communication device, such as wireless telephone, radio telephone or cellular, and any necessary telephone base will be installed in the storage holder.

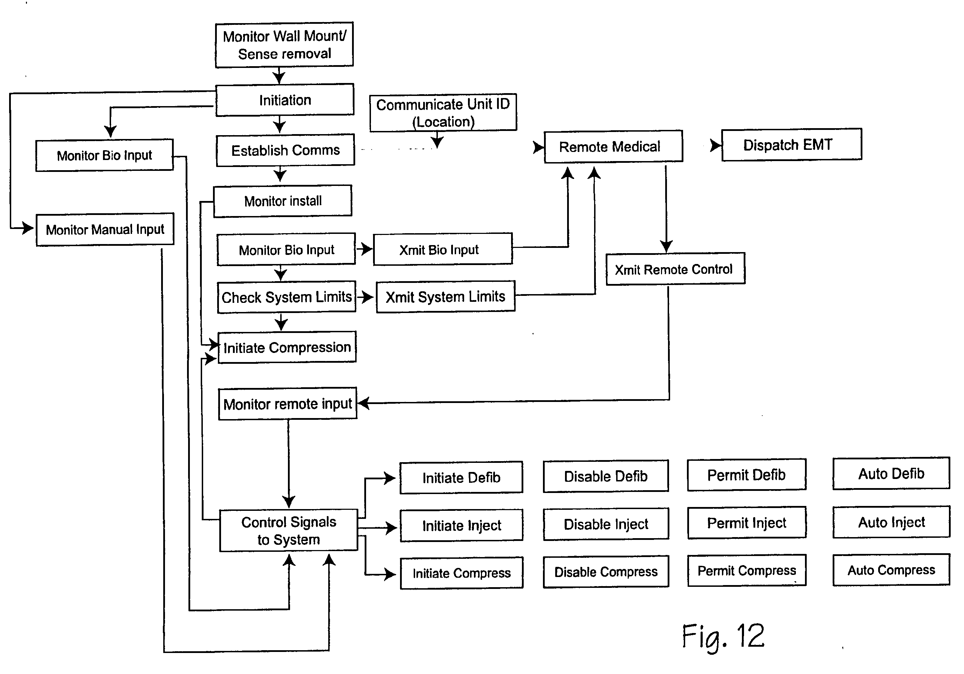

The communications unit and control unit are set up to operate in the following manner, also illustrated in the block diagram of Figure 12. The device may remain mounted in a charging unit for months between use, and will be removed from the charging unit for use. Upon removal of the device from its storage location, a sensor in the control unit senses the removal (through limit switches, magnetic switches, or motion sensors, current sensors in the charging system, or otherwise) and initiates the system, checking functions, energizing a display unit and accomplishing other typical warm-up functions. As a first step, the system initiates a telephone communication with a medical facility through the communications unit. The communication may use any communication medium, whether it be standard telephone lines, cellular telephone system, paging system or radio transmitter. The system may be set up to initiate communications with central medical facility, such as a local 911 emergency system, a nearby hospital or ambulance service, or a central facility staffed with medical personnel trained specifically on the remote use of the device (all generally referred to as medical personnel). Upon establishing communication, the communications unit informs medical personnel of the location or identification of the device (which may be stored in computer memory in the communications unit, or determined through GPS or other such system), and this information can be used to dispatch an emergency medical team to the location of the device. In a simple embodiment which does not require a computer to control the actions of the alert feature, the removal sensor may comprise a limit switch, while the communications module may comprise a simple telephone unit installed in the storage holder together with a tape recorded message, where the operation of the relay in response to removal of the resuscitation device includes initiation of the telephone call to 911 and playback of an alert message providing alert information such as the location of the board. The communications unit may also be provided with an alert button which may be operated by a bystander regardless of the use of the board to summon an emergency team to the location regardless of the condition of the resuscitation device.

Before the emergency medical team arrives, a bystander will place the board under the victim, buckle the compression belt around the victim and apply defibrillation and/or sensing electrodes (or vice versa)(alternatively, sensing electrodes can be included on the inner surface of the compression belt). The system monitors the installation of the belt through signals provided through latching sensors in the buckle. The system monitors biological input, which can comprise monitoring of EKG signals from the EKG electrode patches of the defibrillation module, monitoring EKG signals belt mounted electrodes, monitoring signals from an end-tidal CO₂ monitor from the airway management module, and any other biological signal sensor incorporated into the device. The system can also monitor or respond to manually inputted instruction from the control unit, in order to provide on-site emergency medical personnel with control of the device when they arrive on scene. During operation, the system transmits all available biological information, including EKG signals, blood pressure, end-tidal CO₂ and any other monitored biological parameter to the remote medical facility, and it can also transmit information regarding the configuration of the device, including battery life, system operating limit settings (i.e., whether the system is set for automatic operation, permissive operation, or disabled in any function) so that medical personnel can ensure that the appropriate configuration is in effect.

Communication with the medical facility will allow emergency medical personnel to diagnose the condition of the patient and, through signals sent from the medical personnel to the communications unit, permit, initiate or prohibit certain additional therapeutic ACLS actions. For example, upon diagnosing the EKG conditions which indicate the need for defibrillation, the medical personnel can send a signal to the communications unit which acts upon the control unit to permit manual operation of the defibrillation electrodes by the bystander. The system also provides for application of a defibrillation shock via remote signal from the medical personnel. The device can incorporate the expert system such as the Automatic External Defibrillator. The medical personnel can also communicate other actions, and ensure that certain acts are undertaken by the bystander through the communication system. For example, the medical personnel may communicate verbally with the bystander to ascertain the cause of the cardiac arrest, the proper placement of the oxygen mask, appropriate clearing of the airway, and other information. Where the airway management module is provided with medication such as epinephrine, lidocaine, bretylium or other drugs called for in the ACLS guidelines (or newly proposed drugs such as T3), the medical personnel can instruct by-standers to inject appropriate medication through the airway. Where automatic injectors such as those described in Kramer, Interactive External Defibrillation and Drug Injection System, U.S. Patent 5, 405, 362 (Apr. 11, 1995) are provided, or similar system with non-osseous injectors are provided, the medical personnel can instruct by-standers to inject appropriate medication through these injectors. Where the injectors are provided with means for automatic operation based on measured EKG signals, blood pressure and end-tidal CO2, the medical personnel can send signals to the system to initiate injection by remote control of the medical personnel, permit injection by local control as determined by the expert system, permit injection by by-standers, or prohibit injection by the system or bystanders. For example, the system can be initially set up to forbid any injection. Medical personnel, having diagnosed ventricular fibrillation through the information provided by the communications unit, can send an appropriate signal to permit or initiate injection of epinephrine, and also send a signal to prohibit injection of atropine until called for under the ACLS guidelines. A newly proposed drug T3 can be administered through the airway, into the lungs, as a therapy for cardiac arrest. Controlled injection into the airway can be initiated or prohibited in the same manner. Thus, all actions in the ACLS, including compression, defibrillation, drug injection can be accomplished through the system under the guidance of medical personnel from a remote location, or they may be accomplished through expert systems installed in the control module. Each of these functions is incorporated in a system that automatically initiates communication with medical personnel and informs medical personnel of the location of the device so that emergency medical personnel my be dispatched to the location.

The repeated compression will be initiated upon buckling of the compression belt (automatically) or a switch can be provided for the bystander to initiate compression. The system will continue compression cycles, until de-activated, according to the motor control block diagram of Figure 13. Upon initiation of the system, the control unit will monitor installation of the belt via appropriate sensors in the buckles or through other sensors. When the motor control receives the initiate compression signal from the control unit, the motor is started. The motor is preferably run continuously, rather than stopped and started, to avoid repeated application of startup current and thus conserve battery power. When the motor is up to speed, the clutch is engaged. As a baseline, the clutch is engaged every second for one-half second. This cyclic engagement of the clutch continues repeatedly for five cycles, as recommended by current CPR guidelines, and then is interrupted for a respiration pause, if desired. To avoid excessive drain on the batteries, the motor controller includes a torque sensor (sensing current supply to the motor, for example), and monitors the torque or load on the motor. A threshold is established above which further compression is not desired or useful, and if this occurs during the half second of clutch engagement, then the clutch is disengaged and the cycle continues. The system can monitor the effectiveness of the compression stroke by monitoring the CO₂ content of the victim's exhalant. Where CO₂ content is low, indicating inadequate circulation, the control system increases the torque limit until the CO₂ levels are acceptable (or until the maximum torque of the motor is achieved). This is another example of the device's use of biological signals to control operation of the system. The cycle time and period, number of cycles between respiration pauses, and the torque limit, can be set according to current guidelines, and can also be varied by the remote medical personnel via the remote control capabilities of the control unit.

Thus, while the preferred embodiments of the devices and methods have been described in reference to the environment in which they were developed, they are merely illustrative of the principles of the inventions. Other embodiments and configurations may be devised without departing from the scope of the appended claims.

## Claims

1. A chest compression device (2) comprising:
a belt (3) which is adapted to extend at least partially around the chest of a human;
a rotating spool (12) operatively connected to the belt (3) to constrict the belt about the chest;
a friction liner (5) adapted to be disposed between the belt (3) and the chest of the human when the belt (3) is extended around the chest of the human, wherein said friction liner (5) is adapted to extend substantially completely around the chest of the human, said friction liner (5) permitting the belt (3) to slide freely over said friction liner (5);
an electric motor (14) for rotating the rotating spool (12) ;
a clutch (22) for operatively connecting the motor (14) to the rotating spool (12); and
a controller for repeatedly engaging and disengaging the clutch (22);
wherein the controller is capable of disengaging the clutch (22) while the motor (14) is operating.

2. The chest compression device (2) of claim 1, wherein the belt (3) is adapted to encircle the chest of the human.

3. The chest compression device (2) of claim 1, further comprising:
a torque sensor operatively connected to the motor (14) to sense the torque applied by the motor (14);
wherein the controller is capable of disengaging the clutch (22) when the torque sensed by the torque sensor reaches a specified limit.

4. The chest compression device (2) of claim 3, wherein the torque sensor is a current sensor which is capable of sensing current supplied to the motor (14).

5. The chest compression device (2) of claim 1, further comprising:
a biological parameter sensor for sensing a biological parameter of the human and transmitting a corresponding signal to the controller;
wherein the controller is capable of adjusting the torque at which the clutch (22) disengages based upon the signal corresponding to the sensed biological parameter.

6. The chest compression device (2) of claim 5, wherein the biological parameter sensor is an end-tidal CO₂ sensor which senses CO₂ in the human's exhalant which transmits a signal corresponding to the level of CO₂ in the exhalant to the controller.

7. The chest compression device (2) of claim 5, wherein the biological parameter sensor is a blood oxygen sensor which senses the level of blood oxygen in the human's blood and which transmits a signal corresponding to the level of blood oxygen to the controller.

8. The chest compression device (2) of claim 1, wherein:
the motor (14) is operatively connected to the rotating spool (12) to rotate the rotating spool (12) thereby causing the belt (3) to constrict around the chest of the human; and
said clutch (22) connecting the motor (14) to the rotating spool (12), said clutch (22) being operable to disconnect the motor (14) from the rotating spool (12) while the motor (14) is operating, and being operable to connect the motor (14) to the rotating spool (12) while the motor (14) is running;
wherein said motor (14) and said clutch (22) are operable to alternatingly rotate the rotating spool (12) in a direction causing the belt (3) to constrict around the chest of the human, and permit the rotating spool (12) to spin in a direction causing the belt (3) to loosen around the chest of the human, wherein said alternate rotations of the rotating spool (12) in the direction causing constriction and rotations of the rotating spool (12) in the direction permitting relaxation occur while the motor (14) is running.

9. The chest compression device (2) of claim 1, wherein said friction liner (5) is adapted to extend substantially completely around the chest of the human, said friction liner (5) being not attached to the belt (3) such that the belt (3) is free to slide over said friction liner (5).

10. The chest compression device (2) of claim 1, wherein said friction liner (5) is adapted to extend substantially completely around the chest of the human, said friction liner (5) being separate from the belt (3).

11. The chest compression device (2) of claim 1, wherein said friction liner (5) provides a substantially stationary surface for the belt (3) to slide over as the belt (3) is constricted about the chest by the rotating spool (12).

12. The chest compression device (2) of claim 1, wherein said friction liner (5) is provided in the form of a second belt, said friction liner (5) being adapted to be disposed between the belt (3) and the chest of the human when the belt (3) is extended around the chest of the human, said friction liner (5) being adapted to extend substantially completely around the chest of the human, wherein said friction liner (5) is not operatively connected to the rotating spool (12).

13. The chest compression device (2) of any of claims 9 to 12, wherein the rotating spool (12) is operatively connected to the belt (3) at a midpoint on the belt (3) so that rotation of the rotating spool (12) draws the belt (3) upon the rotating spool (12) from two directions.

## Patentansprüche

1. Eine Brustkompressionsvorrichtung (2), aufweisend:
einen Gurt (3), welcher angepasst ist, um sich zumindest teilweise um die Brust von einem Menschen herum zu erstrecken,
eine Rotationsspule (12), welche betreibbar mit dem Gurt (3) verbunden ist, um den Gurt um die Brust zu ziehen,
einen Reibungseinsatz bzw. eine Reibungseinlage (5), welche angepasst ist, um zwischen dem Gurt (3) und der Brust von dem Menschen angeordnet zu werden, wenn sich der Gurt (3) um die Brust von dem Menschen herum erstreckt, wobei der Reibungseinsatz (5) angepasst ist, um sich im Wesentlichen vollständig um die Brust von dem Menschen herum zu erstrecken, wobei der Reibungseinsatz (5) es dem Gurt (3) ermöglicht, frei bzw. unbehindert über den Reibungseinsatz (5) zu gleiten,
einen Elektromotor (14) zum Drehbewegen der Rotationsspule (12),
eine Kupplung (22) zum betreibbaren Verbinden des Motors (14) mit der Rotationsspule (12) und
eine Steuerung zum wiederholten in Eingriff bringen und außer Eingriff bringen der Kupplung (22),
wobei die Steuerung im Stande ist, die Kupplung (22) während des Betriebs des Motors (14) außer Eingriff zu bringen.

2. Die Brustkompressionsvorrichtung (2) nach Anspruch 1, wobei der Gurt (3) angepasst ist, um die Brust von dem Menschen zu umfassen.

3. Die Brustkompressionsvorrichtung (2) nach Anspruch 1 ferner aufweisend:
einen Drehmomentsensor, welcher betreibbar mit dem Motor (14) verbunden ist, um das Drehmoment, welches von dem Motor (14) aufgebracht wird, abzutasten,
wobei die Steuerung im Stande ist, die Kupplung (22) außer Eingriff zu bringen, wenn das Drehmoment, welches von dem Drehmomentsensor abgetastet wird, einen festgelegten Grenzwert erreicht.

4. Die Brustkompressionsvorrichtung (2) nach Anspruch 3, wobei der Drehmomentsensor ein Stromsensor ist, welcher im Stande ist, den Strom abzutasten, der dem Motor (14) zugeführt wird.

5. Die Brustkompressionsvorrichtung (2) nach Anspruch 1, ferner aufweisend:
einen Biologischer-Parameter-Sensor zum Abtasten eines biologischen Parameters des Menschen und Übermitteln eines entsprechenden Signals an die Steuerung,
wobei die Steuerung im Stande ist, basierend auf dem Signal entsprechend dem abgetasteten biologischen Parameter das Drehmoment anzupassen, bei dem die Kupplung (22) außer Eingriff gebracht wird.

6. Die Brustkompressionsvorrichtung (2) nach Anspruch 5, wobei der Biologische-Parameter-Sensor ein Endexpiratorisch-CO₂-Sensor ist, welcher CO₂ in dem Atem von dem Menschen abtastet und welcher ein Signal entsprechend dem CO₂-Wert in dem Atem an die Steuerung übermittelt.

7. Die Brustkompressionsvorrichtung (2) nach Anspruch 5, wobei der Biologische-Parameter-Sensor ein Blut-SauerstoffSensor ist, welcher den Wert von Blut-Sauerstoff in dem Blut des Menschen abtastet und welcher ein Signal entsprechend dem Wert von Blut-Sauerstoff an die Steuerung übermittelt.

8. Die Brustkompressionsvorrichtung (2) nach Anspruch 1, wobei:
der Motor (14) betreibbar mit der Rotationsspule (12) verbunden ist, um die Rotationsspule (12) zu drehen, wodurch ein Ziehen des Gurts (3) um die Brust von dem Menschen herum verursacht wird, und
die Kupplung (22) den Motor (14) mit der Rotationsspule (12) verbindet, wobei die Kupplung (22) betreibbar ist, um den Motor (14) von der Rotationsspule (12) zu trennen, während der Motor (14) betrieben wird, und betreibbar ist, um den Motor (14) mit der Rotationsspule (12) zu verbinden, während der Motor (14) läuft,
wobei der Motor (14) und die Kupplung (22) betreibbar sind, um abwechselnd die Rotationsspule (12) in einer Richtung zu rotieren, welche ein Zusammenziehen des Gurts (3) um die Brust von dem Menschen herum verursacht, und der Rotationsspule (12) zu ermöglichen, in einer Richtung zu drehen, welche ein Lockern des Gurts (3) um die Brust von dem Menschen herum verursacht, wobei die wechselnden Drehbewegungen von der Rotationsspule (12) in derjenigen Richtung, welche ein Zusammenziehen verursacht, und Drehbewegungen der Rotationsspule (12) in derjenigen Richtung, welche eine Entspannung zulassen, auftreten, während der Motor (14) läuft.

9. Die Brustkompressionsvorrichtung (2) nach Anspruch 1, wobei der Reibungseisatz (5) angepasst ist, um sich im Wesentlichen vollständig um die Brust von dem Menschen herum zu erstrecken, wobei der Reibungseinsatz (5) nicht an dem Gurt (3) befestigt ist, so dass der Gurt (3) frei über den Reibungseinsatz (5) gleiten kann.

10. Die Brustkompressionsvorrichtung (2) nach Anspruch 1, wobei der Reibungseinsatz (5) angepasst ist, um sich im Wesentlichen vollständig um die Brust von dem Menschen herum zu erstrecken, wobei der Reibungseisatz (5) separat von dem Gurt (3) ist.

11. Die Brustkompressionsvorrichtung (2) nach Anspruch 1, wobei der Reibungseinsatz (5) eine im Wesentlichen stationäre Fläche bereitstellt, über die der Gurt (3) gleiten kann, wenn der Gurt (3) von der Rotationsspule (12) um die Brust herum gezogen wird.

12. Die Brustkompressionsvorrichtung (2) nach Anspruch 1, wobei der Reibungseinsatz (5) in der Form eines zweiten Gurts bereitgestellt ist, wobei der Reibungseinsatz (5) angepasst ist, um zwischen dem Gurt (3) und der Brust von dem Menschen angeordnet zu werden, wenn der Gurt (3) sich um die Brust von dem Menschen herum erstreckt, wobei der Reibungseinsatz (5) angepasst ist, um sich im Wesentlichen vollständig um die Brust von dem Menschen herum zu erstrecken, wobei der Reibungseinsatz (5) nicht betreibbar mit der Rotationsspule (12) verbunden ist.

13. Die Brustkompressionsvorrichtung (2) nach einem der Ansprüche 9 bis 12, wobei die Rotationspule (12) an einem Mittelpunkt des Gurts (3) betreibbar mit dem Gurt (3) verbunden ist, so dass eine Rotation der Rotationsspule (12) den Gurt (3) von zwei Richtungen auf die Rotationsspule (12) zieht.

## Revendications

1. Dispositif de compression de poitrine (2) comprenant :
une bande (3) qui est à même de s'étendre au moins en partie autour de la poitrine d'un patient ;
une bobine rotative (12) raccordée en service à la courroie (3) pour resserrer la courroie autour de la poitrine:
une garniture de friction (5) qui est à même d'être disposée entre la courroie (3) et la poitrine du patient lorsque la courroie (3) est étendue autour de la poitrine du patient, dans lequel ladite garniture de friction (5) est à même de s'étendre sensiblement complètement autour de la poitrine du patient, ladite garniture de friction (5) permettant à la courroie (3) de glisser librement sur ladite garniture de friction (5) ;
un moteur électrique (14) pour faire tourner la bobine rotative (12) ;
un embrayage (22) pour raccorder en service le moteur (14) à la bobine rotative (12) ; et
un dispositif de commande pour engager et désengager de manière répétée l'embrayage (22) ;
dans lequel le dispositif de commande est à même de désengager l'embrayage (22) tandis que le moteur (14) est en cours de fonctionnement.

2. Dispositif de compression de poitrine (2) selon la revendication 1, dans lequel la courroie (3) est à même d'enserrer la poitrine du patient.

3. Dispositif de compression de poitrine (2) selon la revendication 1, comprenant en outre :
un capteur de couple connecté en service au moteur (14) pour détecter le couple appliqué par le moteur (14) ;
dans lequel le dispositif de commande est à même de désengager l'embrayage (22) lorsque le couple détecté par le capteur de couple atteint une limite spécifiée.

4. Dispositif de compression de poitrine (2) selon la revendication 3, dans lequel le capteur de couple est un capteur de couple qui peut détecter le courant fourni au moteur (14).

5. Dispositif de compression de poitrine (2) selon la revendication 1, comprenant en outre :
un capteur de paramètre biologique pour détecter un paramètre biologique du patient et transmettre un signal correspondant au dispositif de commande ;
dans lequel le dispositif de commande est à même d'ajuster le couple au niveau duquel l'embrayage (22) se désengage sur la base du signal correspondant au paramètre biologique détecté.

6. Dispositif de compression de poitrine (2) selon la revendication 5, dans lequel le capteur de paramètre biologique est un capteur de CO₂ de fin d'expiration qui détecte le CO₂ dans l'exhalaison du patient et transmet un signal correspondant au niveau de CO₂ dans l'exhalaison au dispositif de commande.

7. Dispositif de compression de poitrine (2) selon la revendication 5, dans lequel le capteur de paramètre biologique est un capteur d'oxygène sanguin qui détecte le niveau d'oxygène sanguin dans le sang du patient et transmet un signal correspondant au niveau d'oxygène sanguin au dispositif de commande.

8. Dispositif de compression de poitrine (2) selon la revendication 1, dans lequel :
le moteur (14) est connecté en service à la bobine rotative (12) pour faire tourner la bobine rotative (12) de manière à amener la courroie (3) à se resserrer autour de la poitrine du patient ; et
ledit embrayage (22) connecte le moteur (14) à la bobine rotative (12), ledit embrayage (22) pouvant être actionné pour déconnecter le moteur (14) de la bobine rotative (12) tandis que le moteur (14) est en cours de fonctionnement et pouvant être actionné pour connecter le moteur (14) à la bobine rotative (12) tandis que le moteur tourne ;
dans lequel ledit moteur (14) et ledit embrayage (22) peuvent être actionnés pour faire tourner en alternance la bobine rotative (12) dans un sens qui amène la courroie (3) à se resserrer autour de la poitrine du patient et permettent à la bobine rotative (12) de tourner dans un sens permettant à la courroie (3) de se desserrer autour de la poitrine du patient, dans lequel lesdites rotations alternées de la bobine rotative (12) dans le sens permettant le resserrement et les rotations de la bobine rotative (12) dans le sens permettant un relâchement se produisent tandis que le moteur (14) tourne.

9. Dispositif de compression de poitrine (2) selon la revendication 1, dans lequel ladite garniture de friction (5) est à même de s'étendre sensiblement complètement autour de la poitrine du patient, ladite garniture de friction (5) n'étant pas fixée à la courroie (3) si bien que la courroie (3) est libre de glisser sur ladite garniture de friction (5).

10. Dispositif de compression de poitrine (2) selon la revendication 1, dans lequel ladite garniture de friction (5) est à même de s'étendre sensiblement complètement autour de la poitrine du patient, ladite garniture de friction (5) étant séparée de la courroie (3).

11. Dispositif de compression de poitrine (2) selon la revendication 1, dans lequel ladite garniture de friction (5) offre une surface sensiblement stationnaire pour la courroie (3) pour permettre à celle-ci de glisser sur cette surface lorsque la courroie (3) est resserrée autour de la poitrine par la bobine rotative (12).

12. Dispositif de compression de poitrine (2) selon la revendication 1, dans lequel ladite garniture de friction (5) se présente sous la forme d'une seconde courroie, ladite garniture de friction (5) étant à même d'être disposée entre la courroie (3) et la poitrine du patient lorsque la courroie (3) est étendue autour de la poitrine du patient, ladite garniture de friction (5) étant à même de s'étendre sensiblement complètement autour de la poitrine du patient, ladite garniture de friction (5) n'étant pas connectée en service à la bobine rotative (12).

13. Dispositif de compression de poitrine (2) selon l'une quelconque des revendications 9 à 12, dans lequel la bobine rotative (12) est connectée en service à la courroie (3) en un point central sur la courroie (3) de sorte que la rotation de la bobine rotative (12) tire la courroie (3) sur la bobine rotative (12) dans deux sens.
